# EUROPEAN PATENT APPLICATION

(11) **EP 3 839 962 A1**
(43) Date of publication of application: **23.06.2021**
(21) Application number: 19217751.7
(22) Date of filing: 18.12.2019
(51) Int. Cl.: G16H 20/10

(54) **SYSTEM AND METHOD FOR MONITORING ADHERENCE TO DRUG REGIMEN**

(71) Applicant: Healthbeacon Limited, D12 WD85 Dublin (IE)
(72) Inventor: Joyce, James, Dublin, D12 WD85 (IE); Daly, Kieran, Dublin, D12 WD85 (IE); Shattock, Richard, Dxublin, D12 WD85 (IE)
(74) Representative: Hanna Moore + Curley

(57) **Abstract**

A system and method for monitoring patient medical adherence of a user of an associated medicament container. The medicament container is configured to receive medicament deposits and/or to dispense medicament, and to transmit medicament information each time the medicament container is operated by the user. A medical adherence monitoring platform is configured for receiving, via a communication link, the medicament information from the at least one medicament container, the adherence monitoring platform comprising a classification engine configured for classifying the medicament information according to a prescribed medicament schedule associated with the user and/or dosage regime. The classified medicament information being indicative of the medical adherence of the user to their prescribed medicament schedule and/or to a prescribed dosage regime. The classified medicament information being stored in an adherence database.

## Description

### Field

The present application relates generally to medical adherence, and more specifically to a system and method for monitoring patient medical adherence behaviour.

### Background

Chronic diseases such as diabetes, allergies, and cardiovascular or degenerative diseases have increased their prevalence in the past decades. Such chronic diseases often require long-term treatment typically in the form of self-administered medications. In order to produce the desired therapeutic effect, the patients are required to follow a regimen or a schedule prescribed or recommended by a healthcare provider. The extent to which a person's behaviour in taking medication corresponds with agreed recommendations from a healthcare provider is referred to as "medical adherence". Although medical adherence is extremely important for effective treatments, research and surveys have shown that a large proportion of patients fail to monitor medical adherence. The lack of adherence to prescribed medications and therapies is referred to as "medical non-adherence".

Among various forms of self-administered medications, those requiring self-injection are particularly challenging in ensuring medical adherence due to complexity in medical regimen and difficulty in establishing good injection habits. Non-adherence to injectable medication is thus a constant issue. This puts increased strains on healthcare services in the long term. Healthcare professionals are often not aware of their patient's adherence levels and rely on patient's feedback as being honest. As patients may not want to disappoint their doctor, they often bend the truth on the level of their adherence. Therefore, adherence assessment based on self report is hard to verify accurately.

Many methods have been developed hoping to address the issue of medical non-adherence. Typically, when a patient is prescribed a medication, e.g. injectable medication, for a chronic disease it is usually accompanied by a patient support programme to assist the patient with information on their disease and treatment. Such patient support programme typically involves providing nurse visits, training the patient on how to administer their medication, and providing support materials. Helplines and tools can be available to address queries around the disease and medication.

Conventional approaches for assessing and improving patient's medical adherence mainly involve, for example, using network applications or social networking platforms to improve the quality and convenience of doctor-patient communications. US 2015/0216413 A1 discloses systems, devices, and methods for analysing and enhancing patient health using a social networking platform interconnecting patients with healthcare providers.

In the context of self-injected medication, PCT/US2016/042733 discloses a medical adherence device, in particular a sharps bin which allows accurate detection of the number of sharps disposed in a sharps bin. The device facilitates healthcare provider to obtain accurate information as to whether the patients are complying with their medical treatments. Intervention may be initiated by the healthcare provider to correct the patient's behaviour if necessary. However, there remains an issue of effectively preventing non-adherence behaviour to improve patient adherence level.

In summary, existing approaches are either expensive to maintain or ineffective for maintaining a long-term medication adherence. They either rely on patients being proactively involved in the process to seek assistance in order to enjoy the benefits or fails to prevent non-adherence behaviour. Furthermore, conventional approaches often overlook individual differences between patients. For example, different patients may require different levels of assistance or interventions from the healthcare provider in order to maintain an acceptable level of medical adherence.

There exists a strong demand for a more effective method and system for assisting patient to prevent non-adherence to improve their medical adherence level, particularly in administering self-injected medications.

### Summary

These and other problems are addressed in accordance with the present teaching by a method and system for assisting medical adherence of a user of a smart sharps bin configured for receiving deposits of sharps of administered injected medication.

Accordingly, the present invention provides a system and a method as detailed in independent claims. Advantageous embodiments are provided in the dependent claims.

A system and a computer program which are respectively configured to carry out the method may also be provided.

The present invention offers an effective solution to improve adherence level for patients following long-term self-medication regimens. The medical adherence system of the present invention is configured to monitor medical adherence of connected users according to their corresponding medicament schedule based on their past medicament administration behaviour. In the case, where there is a deviation from a user's corresponding medicament schedule, the adherence monitoring system of the present invention is configured to send one or more reminders to prompt the user to take the medication on time, and/or adjust his/her dosing to meet the needs of the treatment. In this way, the medicament adherence of a patient may be improved via timely interventions.. If necessary, the service may initiate one or more other forms of interventions to prompt the user to take a prescribed medication. For example, manual interventions may be requested by the adherence monitoring system by sending data to an online dashboard where a nursing team may observer a patient's progress with a prescribed medical treatment. The present inventio offers an effective solution for supporting patients achieve a greater medication adherence at home. For example, data collected from a smart sharps bin configured to record and timestamp deposits of spent sharps may provide insights into patient self-injection behaviour and allows the identification of patterns within individual patients, patient groups, geographical populations, prescribed medicament, and other type of information. The adherence monitoring system of the present invention may comprise an ensemble predictive model to improve the accuracy of predictions made on patient medicament adherence behaviours which may allow enable more accurate and timely notifications to be issued resulting an improvement of the patient adherence performance.
The system of the present invention may further help users to manage complex medication regimes involving one or more medicament through the generation of timely prompts and reminders. In this way, the system of the present invention simplifies the administration process involved in a medicament schedule while providing validated data on the medicament administration behaviour of a user to health care providers. The ability to record the time of disposal and reconcile this back to the patient's prescription schedule allows the calculation of an adherence score that may be used to inform patients on their adherence performance and alert the healthcare providers,

### Brief Description of the Drawings

Figure 1 shows an exemplified implementation of a system for assisting medical adherence in accordance with embodiments of the present invention.
Figure 1A shows a schematic of an exemplified sharps bin according to embodiments of the present invention.
Figure 1B shows an exemplified model architecture of an ensemble predictive model according to embodiments of the present invention.
Figure 2 shows a process flow of an exemplary method for monitoring patient medical adherence behaviour in accordance with embodiments of the present invention.
Figure 3 shows a process flow of an exemplary method for generating predictive model in accordance with embodiments of the present invention.
Figure 4A shows a process flow of an exemplary method for predicting a user's adherence behaviour in accordance with embodiments of the present invention.
Figure 4B shows a resulting medical adherence prediction using the exemplary method of Figure 4A.
Figure 5 shows a process flow of an exemplary method for monitoring the medical adherence of a user in accordance with embodiments of the present invention.
Figure 6 shows a process flow of an exemplary method for validating a predictive model in accordance with embodiments of the present invention.

### Detailed Description

The present invention will now be exemplified with reference to the accompanying drawings.

Throughout the following discussion, numerous references will be made regarding servers, services, interfaces, portals, platforms, or other systems formed from computing devices. It should be appreciated that the use of such terms is deemed to represent one or more computing devices having at least one processor configured to execute software instructions stored on a computer readable medium. For example, a server can include one or more computers operating as a web server, database server, or other type of computer server in a manner to fulfil described roles, responsibilities, or functions. One should further appreciate the disclosed computer-based algorithms, processes, methods, or other types of instruction sets can be embodied as a computer program product comprising computer readable media storing instructions that cause a processor to execute the disclosed steps. One should appreciate that the systems and methods described herein involve interconnected networks of hardware devices configured to receive data using receivers, transmit data using transmitters, and transform electronic data signals.

The following discussion provides many exemplary embodiments of the inventive subject matter. Although each embodiment represents a single combination of inventive elements, the inventive subject matter is considered to include all possible combinations of the disclosed elements. Thus, if one embodiment comprises elements A, B, and C, and a second embodiment comprises elements B and D, then the inventive subject matter is also considered to include other remaining combinations of A, B, C, or D, even if not explicitly disclosed.

A number of terms used in describing various embodiments of the present invention may be used synonymously and interchangeably. For example, the term "medicament" may be used synonymously with "medicament item", "medicament product", "medicine", "medication", or "drug". The term "medicament container" may be used to refer to any container suitable for containing and receiving and/or dispensing medicament or medicament items. The term "medical adherence" may be used synonymously with "medical compliance". The term "patient" may be used synonymously with "user" or "user of the sharps bin". The term "spent medical sharps" may be used synonymously with "medical sharps", "sharps", "needles", "syringes", or "hypodermics". The term "deposit" may be used synonymously with "dispose", "disposal", or "drop". As used herein, and unless the context dictates otherwise, the term "coupled to" or "connected to" is intended to include both direct coupling or connection (in which two elements that are coupled or connected to each other contact each other) and indirect coupling or connection (in which at least one additional element is located between the two elements). Therefore, the terms "coupled to", "coupled with", "connected to" and "connected with" are used synonymously.

For simplicity and clarity of illustration, reference numerals may be repeated among the figures to indicate corresponding or analogous elements. Numerous details are set forth to provide an understanding of the examples described herein. The examples may be practiced without these details. In other instances, well-known methods, procedures, and components are not described in detail to avoid obscuring the examples described. The description is not to be considered as limited to the scope of the examples described herein.

According to the present invention a system for monitoring patient medical adherence behaviour is provided. The system comprises at least one medicament container associated with a user. The medicament container may be configured to receive medicament deposits and/or to dispense medicament. For example, the medicament container may be a sharps bin or a similar container that is configured to receive sharps deposits from a user. Furthermore, the medicament container may be configured to store and/or dispense medicament products to the user as part of their medical treatments e.g. pills, syringes, and the like. The medicament container may be configured to transmit medicament information each time the medicament container is operated by the user, e.g. for the retrieval and/or disposal of medicament items from the container. The system may comprise a medical adherence monitoring platform or service. The medical adherence monitoring platform 102 may be configured for receiving, via a communication link, the medicament information from the at least one medicament container. The adherence monitoring platform may comprise a classification engine configured for classifying the medicament information according to a prescribed medicament schedule associated with the user. For example, the user may be associated with a medical treatment, which requires medication to be taken at a prescribed medicament schedule. The classified medicament information may be indicative of the medical adherence of the user to their prescribed medicament schedule. The system may comprise an adherence database configured for storing the classified medicament information of the user.

The system may comprise a predictive model trained using the classified medicament information stored in the database. The system may further comprise at least one processor configured to analyse the medical adherence behaviour of the user. The analysis may be achieved by retrieving from the database classified medicament information associated with the user and inputting the retrieved classified medicament information to a predictive model to generate a prediction output indicating whether an expected medicament deposit and/or dispensation would be made by the user within a timeframe specified in their medicament schedule. The processor may be configured for issuing a first communication to at least one user device 110 when the prediction output indicates a deviation from the user's medicament schedule.

According to some embodiments, the system is configured for monitoring patient medical adherence behaviour in administering self-injected medications. Accordingly, the at least one medicament container is a sharps bin configured to receive sharps deposits by an associated user, the at least one sharps bin being configured to transmit sharps deposit information each time a sharps deposit is made by the associated user.

There is also provided a method 200 for monitoring patient medical adherence in accordance with an embodiment of the invention. The method may comprise the steps of providing at least one medicament container associated with a user 202. The medicament container may receive 204 medicament deposits and/or dispense medicament or medicament items. The medicament container may transmit 206 medicament information each time the medicament container is operated by the user. The method may comprise receiving 208 at an adherence monitoring platform 102, via a communication link, the medicament information from the at least one medicament container. The adherence monitoring platform may be configured to perform the steps of classifying 210 by means of a classification engine 101 the medicament information according to a prescribed medicament schedule associated with the user. The classification engine 101 may be configured to compare the timestamp of the medicament information to that of the prescribed medicament schedule and accordingly classify the medicament information. The classified medicament information may be indicative of the medical adherence of the user to their prescribed medicament schedule. For example, the medicament information may be classified as being late, on-time, early, or in any preferred classification format e.g. score, and the like. The method may comprise storing 212 at a database the classified medicament information of the user.

According to some embodiments, the method may comprise the step of training a predictive model using the classified medicament information stored in the database and analysing by at least one processor configured to analyse the medical adherence behaviour of the user. The analysis may comprise retrieving from the database classified medicament information associated with the user and inputting the retrieved classified medicament information to a predictive model to generate a prediction output indicating whether an expected medicament deposit and/or dispensation would be made by the user within a timeframe specified in their medicament schedule. The processor may be configured for issuing a first communication to at least one user device 110 when the prediction output indicates a deviation from the user's medicament schedule.

The system and the method may be adapted to generate reminders or other types of intervention acts if the prediction output indicates that there will be a deviation from the user's medicament schedule e.g. the user would make the next deposit late. The system and the method may also be adapted to generate alerts in the event that overdosing and/or underdosing is detected or predicted. For example, if the user operated the medicament container, e.g. a sharps bin to deposit a dispensed sharp or a pill container to dispense pills, more frequently that required by the medicament schedule, the system may classify such behaviour as "overdose". Similarly, if the user operates the medicament container infrequently, the user behaviour may be classified by the system as "underdosing", and accordingly issue notifications and alerts to the user for a corrective dose schedule Accordingly, communications as described in connection with the various embodiments described herewith may be triggered to alert the user.

While the present invention has been exemplified with references to a medicament container configured as a sharps bin system, it should be understood that the various configurations and operations described herewith may be adapted to suit various types of medicament containers other than sharps bins. For example, the containers may be various types of containers adapted for receiving spent medicine bottles, tubes, etc. The medicament containers may also be adapted for dispensing medicament or medications to be administered by patients as part of their medical treatment. The containers may be a stationary container located in patient's home. The containers may also be portable containers such as those attachable to user devices 110. An example of such a portable container is a mobile phone case configured to receive and/or dispense medicament items. The mobile phone case may be communicatively coupled to the mobile phone so that each time the container is operated by the user, the mobile phone can transmit medicament information to a server.

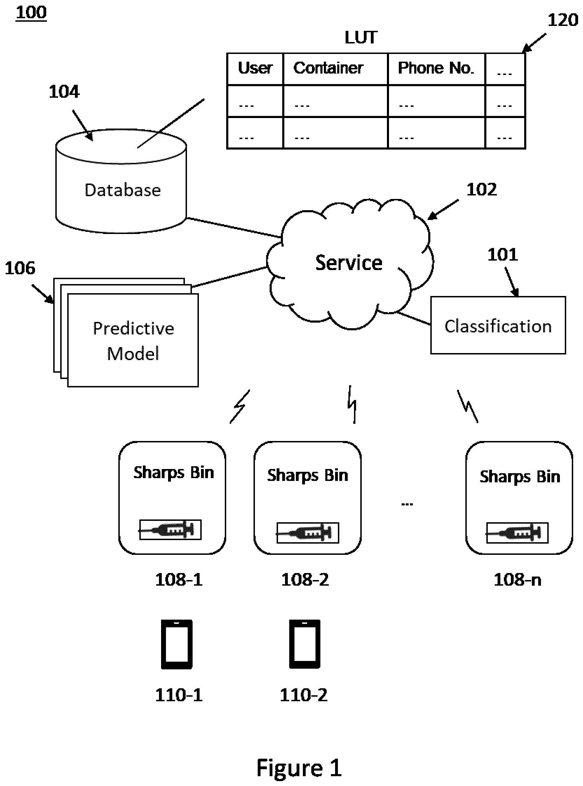

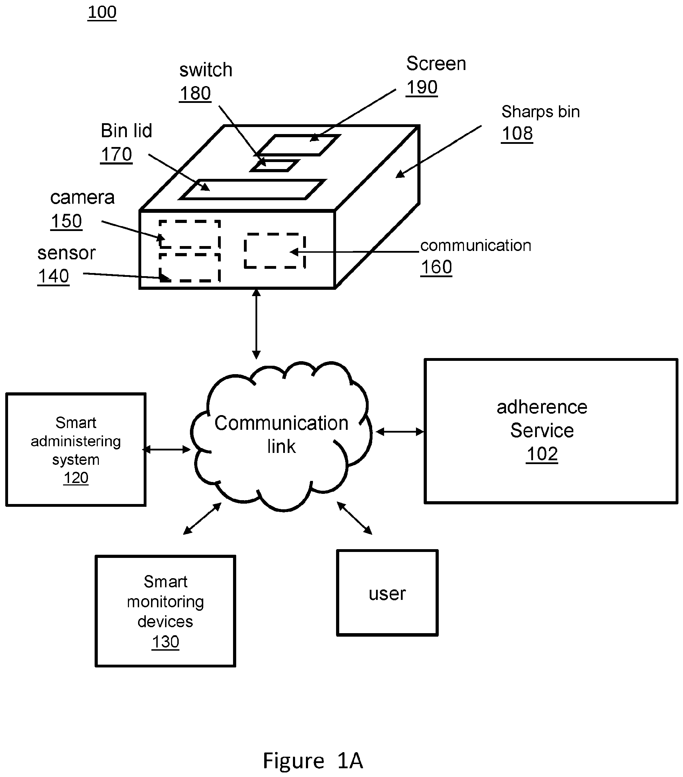

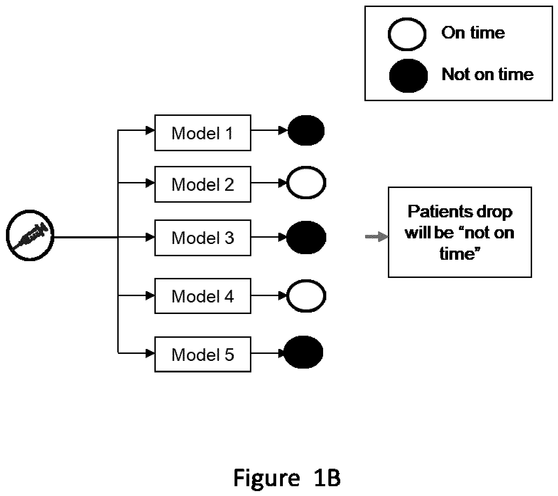

Figure 1 shows a schematic of a system 100 for monitoring or assisting with medical adherence in accordance with the embodiment of the medicament containers being sharps bins. The system 100 includes one or more electronic devices providing a medical adherence service 102, also referred to as a medical adherence monitoring platform 102. The medical adherence monitoring platform may include a classification engine 101. The classification engine 101 may be configured to compare the timestamp of the medicament information to that of the prescribed medicament schedule and accordingly classify the medicament information. The system 100 also includes an adherence database 104, a predictive model 106, and one or more sharps bins 108 each optionally associated with at least one user computing device such as a user device 110 (i.e. a mobile phone). Each component typically includes at least one processor, a data storage system (including volatile memory or non-volatile memory or other data storage elements or a combination thereof), and at least one communication interface. The components are connected or coupled through one or more networks. The network may be based on any suitable technology and may operate according to any suitable protocol and may include wired or wireless networks, or any combination thereof. For example, the network may be one or more of a local area network, a wide area network, Ethernet or the Internet.

The adherence service 102 or simple the service 102 includes applications or web-based platforms providing medical adherence assistance implemented through one or more servers. The one or more servers may be one or more computers operating as a cloud server, web server, database server, or other type of computer server. In a preferred embodiment, the service 102 is a cloud-based medical adherence monitoring platform 102.

The adherence database 104 is coupled to the service through the network. The adherence database, or simply the database 104, contains information related to a plurality of users' past adherence behaviour for taking scheduled medications. For example, the adherence database 104 may store sharp deposit information received from a sharps bin that have been classified by means of a classification engine 101 to indicate the medical adherence of the user to their prescribed sharp deposit schedule. The classification engine may be configured to compare the timestamp of the medicament information to that of the prescribed medicament schedule and accordingly classify the medicament information. The medications include but are not limited to injectable medications. For example, the database 104 may contain information on the user's past or historical sharps deposit behaviour such as whether each past deposit is made on time, early, late, or not made at all (missed). The database 104 may contain information relating to each user's prescription including recommended or scheduled medical regimen. The database 104 may further contain information such as user location, user mobile phone number, and/or SMS time, etc. The database 104 may further contain information on user age, gender, diagnosis, etc. The various data contained in the adherence database may be stored in one or more lookup tables 120.

The database 104 may be stored in one or more storage devices such as database servers. The database may be distributed across multiple storage devices connected to one another via a communication network.

The predictive model 106 is configured to predict a user's adherence behaviour. For example, the predictive model 106 may be trained using the classified sharp deposit information stored in the database 104 to predict the user medical adherence behaviour for future sharps deposits. According to an embodiment of the present invention, the predictive model 106 is an ensemble predictive model based on ensemble learning, a simplified model structure of which is shown in Figure 1B as an example. An ensemble predictive model uses multiple predictive models or algorithms, each known as a base model, to obtain a better predictive performance than could be obtained from any of the constituent base model alone. Examples of ensemble predictive models using ensemble learning which are suitable for implementing the various embodiments of the present invention include XGBoost, Random Forest, etc. Although an ensemble predictive model is commonly considered to produce better prediction result, it should be noted that a predictive model based on a single model or algorithm may nevertheless be employed for implementing the present invention for various reasons. For example, a single model based predictive model may save computing resources, increase computing speed, and reduce power consumption. For simplicity and clarity of illustration, the term "predictive model" is used herewith to refer to either a single model or an ensemble model based predictive model where applicable.

According to an embodiment, the predictive model 106 is generated based on training one or more machine learning models. According to another embodiment, the predictive model 106 may be based on other suitable prediction-making algorithms. In the event of a machine-learning-based predictive model, a non-exhaustive list of predictive models can be found in US 2006/0271407 A1.

Figure 1B shows a simplified model architecture of an ensemble predictive model according to an embodiment of the invention. The ensemble predictive model shown in Figure 1B contains five base models. Each base model is configured to produce a prediction result and majority voting is used to select a final predictive result. The five base models may be selected from, for instance, seven models which are validated with an existing dataset from the database. The five best performing models are selected to form the ensemble model of Figure 1B. Although five base models are employed in this example, any number of base models may be employed to constitute an ensemble predictive model. In some embodiments, the five selected models are Extra Trees Classifier, Random Forest, XGBoost, Gradient Boosting, and Multilayer Perception.

The one or more sharps bins (108-1, 108-2 ... 108-n) are configured to receive and store spent medical sharps. According to an embodiment, each sharps bin is adapted to receive deposits of spent medical sharps such as syringes and needles. The sharps bins may also be adapted to receive other types of medical wastes such as spent medicine bottles, tubes, containers, etc.

In addition to the components and features that will be appreciated from the discussion herein, the sharps bins 108 may include features and components in accordance with those described in our earlier published patent application, PCT/US2016/042733.

According to an embodiment, each sharps bin 108 has an on-board processor or computing device (not shown). The computing device may include machine readable instructions and data that pertain to the user's unique injection schedule. The computing device may be configured to record a deposit event following administration of medication. The computing device may be configured to record an image of a received sharp and timestamp the received sharp to allow a reconciliation of usage of the sharp's bin with a prescription schedule. Instead of an on-board computing device, an external computing device operatively connected to the sharp bin may be employed.

According to an embodiment of the present invention, each sharps bin 108 is associated with a user. The association may be achieved by associating a sharps bin 108 with a user's computer device 110 such as a mobile phone, a personal computer, and/or a personal digital assistant. Each sharps bin may include an identification device such as a Bluetooth device, a near field communications (NFC) device, a bar code, a QR code, a radio-frequency identification (RFID) device, and/or other device. In one embodiment, once a sharps bin 108 is associated with a user, the sharps bin may obtain access to various information related to the user. Such information may include the user's name, age, gender, medical prescription, medical regimen, etc. According to a further embodiment, a sharps bin 108 may be configured to support association with multiple users. Each sharps bin 108 may include a SIM to support wireless communication with the medical adherence service 102.

According to an embodiment, the sharps bin 108 may include a global positioning system (GPS) device to enable monitoring and tracking of the sharps bin's location. The sharps bin 108 will typically include a user interface (not shown) and an input module to allow the user to input information or instructions. The received information may be locally stored or immediately transmitted to a remote server such as the database 104.

A sharps bin is typically designed as a cuboid and comprises a few main parts as shown in the schematic diagram in Figure 1. According to an embodiment of the invention, each sharps bin 108 includes at least one receptacle arranged to receive a deposit of at least one sharp each time. The received sharps are stored in a housing of the sharps bin 108. The sharps bin 108 has one or more sensors or detectors located in the sharps bin 108. The one or more sensors 140 are configured to detect a deposit of at least one sharp made into the sharps bin each time. The sharps bin 108 may also has one or more cameras 150 configured to capture one or more images of the detected at least one sharp. The detected deposit of at least one sharp is referred to herewith as an actual deposit or a ground-truth deposit. In one embodiment, the one or more sensors 140 are configured to detect a deposit of a single sharp each time. In another embodiment, the one or more sensors are configured to detect a deposit of more than one sharp each time. In an embodiment where the one or more sensors 140 are adapted to detect more than one sharp each time, the one or more sensors 140 may be configured to determine the number of the sharps. In the embodiments where the sharps bin 108 is adapted to receive various types of medical wastes, the one or more sensors 140 is configured to identify one or more specific types of medical waste among the various types of medical wastes. The one or more sensors 140 may be implemented based on a mechanical sensor, an optical sensor, an electronic sensor, or any combination thereof. In fact, any suitable sensor which enables detecting and/or identifying a deposit of at least one sharp may be employed to facilitate extracting required information on the sharp deposit. As an example, the one or more sensors 140 may include a camera 150 for capturing images of the sharp deposit. Alternatively, the one or more sensors and the camera may be two separate units. In some embodiments, the sharps bin may employ a sensor assembly with a plurality of sensors of the same or different types. Such a sensor assembly can improve sensing accuracy or can be configured to provide additional information or granularity on the nature of the sharps deposit.

Figure 1A shows an exemplary embodiment of a sharps bin connected to a cloud adherence service 102. The uppermost surface contains an LCD screen 190, a button and an integrated sharps bin lid 170. When a patient starts a treatment programme suitable for at-home medication through injection, a smart sharps bin 108 is dispatched to the patient, pre-programmed with their personal medication schedule information which includes information such as the scheduled start date, injection frequency, treatment end date and required injection location (area of body). When an injection is due, the blue lights on the LCD screen 190 light up to remind the patient. The right zone of the LCD screen 190 shows the adherence score of the patient: initialized as 0% at the very beginning of the treatment, the score increases or decreases according to patient's adherence rate over a given period to encourage the patient to stay on track with their medication. When the patient is ready to inject, they turn on the device by pressing the button and administer the medication at the injection location shown on the LCD screen 190. After injection, the patient disposes of the used injector or sharps by pushing it through the bin lid 170 (a traditional hazardous sharps bin sits inside the smart sharps bin).

The sharps bin 108 may have an image recognition unit including for example a micro-camera (150). The image recognition unit may be equipped with a storage media such as an SD card for temporary image storage. The SD card may be fitted slightly below the bin lid 170. The image recognition unit may be configured to take pictures of each object as it falls into the bin. The one or more sensor 140 inside the bin detects the received object. The detected object is timestamped for example when the object enters the bin and the data is recorded (i.e. in CSV format). The images and the recorded data files for each drop are then uploaded to the storage server (i.e. a cloud server) via a communication module 160 such as a cellular module. The images and data files may be transported to an API that can be adopted as an Internet of Things gateway between the sharps bin 108 and the cloud platform 102. The unstructured dataset may be uploaded to a storage service for long-term storage while the dataset saved as CSV files may be migrated to a distributed relational database service for reliable, secure and scalable object storage through a web service interface. Complimentary to the sharps bin data, a web application may be developed where patients need to register before they start the treatment. This web app collects limited personal data such as age, gender, country, hospital ID, medication type and frequency and saves it in the web dashboard as a shared resource for the patient and their healthcare support team.

The one or more sensors 140 typically include a processing unit in communication with a timer and configured to timestamp the received sharp deposit. Typically, the date and/or time of when the sharp is deposited is recorded. The time-stamped information may be stored locally on a storage device located in the sharps bin or transmitted to a remote storage device such as a cloud-based server.

According to embodiments of the present invention, the adherence monitoring system 100 of the present invention may be connected via a communication link to a range of smart medicament administering systems 120, and/or smart monitoring devices that collect and provide additional medicament information, which may be used by the prediction model to determine the adherence of the user to a prescribed medicament schedule and/or dosage regime. The additional data points may be used by the classification engine of the adherence service 102 to classify the patient medicament administration behaviour. For example, the classification engine may classify medicament information received from the different connected device as being late, early, missed, overdosing, underdosing, and the like. The prediction algorithm, based on the classification assigned to the medicament information may generate recommendation and/or notifications to the user, and/or healthcare provider.. According to embodiments of present invention, the adherence monitoring system 100 may determine variations in drug/serum levels within the patient body. For example, with the use of a connecting medicament container 108, e.g. a sharps bin, it is easy to assess whether the patient has deposited their injectable medication in accordance with a prescribed injection schedule. Therefore, it may be assumed that a substance steady-state in the patient's body may be reached with regular administration of the medicament. If the patient varies from the prescribed schedule, e.g. by injecting before (Early) or after (Late) that required then the serum/drug concentration in the body of the patient may change. In such an instance the adherence monitoring system 108 may recommend an intervention or alert the patient that an underdose has been detected, which may necessitate to adjust the medicament schedule until the concentration of the serum/drug reaches the desired levels. Patients demonstrating early, late or missed doses have effectively altered the half-life of the drug. Therefore, the adherence monitoring system offers an effective solution for improving the effectiveness of the prescribed treatment regime.

According to embodiments of the present invention, the adherence monitoring system 100 may comprise a smart monitoring device 130 for measuring patient body parameters that may influence the serum/drug concentration. For example, the adherence service 102 may be connected to a weighing scale, which is configured to collect and transmit weight-based a weight-based data points to the adherence service, which may be used to determine a change in a patient's body surface area, total body weight or lean body weight. Variation in the weight-based data points influences the drug/serum levels in the patient's body. For example, a patient who has gained weight and maintains the prescribed injection schedule is effectively underdosing. Similarly, a patient who has lost weight and maintains a fixed dose regimen may be potentially be overdosing on their medication.
The present invention enables for the providing an effective solution for measuring the theoretical drug/serum level based on either weight or dose administration fluctuations. As the data points are fed into the adherence service 102 and analysed through an algorithm function, the system may provide recommendations or notifications/alerts to the patients should a deviation from the predicted baseline level be detected.

According to embodiments of the present invention, the adherence monitoring system 100 may comprise smart drug administering systems 120, such as smart (connected) autoinjectors, smart syringes, smart pumps or other smart systems that administer medications. The smart drug administering systems 120 may be configured to collect additional data points that may be processed and analysed by the the adherence service platform 102 to enhance the prediction of patient behaviour and accurate generation of notification. For example, the system 100 through a Smart sharps container 108 may connects with the Smart autoinjector pen. The system 100 draws down the medication information from the autoinjector e.g. medication name, concentration, batch number, this information along with the actual volume of administered drug and hence concentration is analysed in the adherence service 102 along with administration time, and/or other information such as the patient's weight obtained through digital scales 130. In the adherence service 102, the algorithm may be provided with details of a pharmacological profile of the specific drug which includes values around half life and clearance rates based on clinical trial or scientific evidence. Based on the information provided the algorithm may accurately predict drug/serum concentration levels in the patient's body and accordingly issue recommendations to the patient.

In another embodiment, the connected sharps container 108 e.g. in the home setting, may be enabled to measure through a blood test the concentration of drug in the system, or allow the user to provide information obtained from a blood test. The system 100 may analyse at the adherence service 102 data obtained from different sources e.g. drug information data, time of administration, weight measurement and drug/serum levels, to generate appropriate recommendations to the patient, caregiver or Healthcare Professional based on real time data. In such a scenario a patient who is maintaining a good habitual injection routine but who's weight has increased may be alerted that of a possible medication underdose. In such a case the system 100 may recommend that the patient adjusts the dosage.

### Generating database

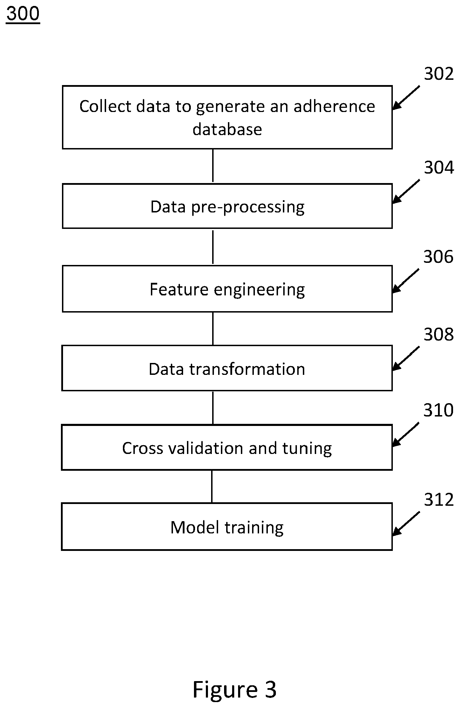

A process flow 300 showing an exemplary process associated with generating a predictive model in accordance with some embodiments of the invention is shown in Figure 3.

At 302, an adherence database 104 is generated by detecting and recording data of a plurality of users relating to their past adherence behaviour in taking prescribed medications including self-injected medications. For example, historic sharp/hypodermic disposal data collected from patients may be used as a primary variable for adherence prediction. Using a large dataset with 4,609 participants and 133,210 historic sharps drops record over a three-year period, a patient adherence predictive model can be developed. The detecting and recording may be effected by communicating with respective sharps bins associated with a plurality of users who may or may not be active users of a particular regimen. For example, information on medical sharp deposit behaviour of a plurality of users may be collected over the past few years. By way of illustration, when a user deposits a medical waste such as a spent sharp in a patient monitoring unit such as a medical waste collection unit (i.e. a sharps bin in accordance but not limited to a sharps bin described herewith), the unit captures and records various information. Data information is captured during each of the deposit opportunities for all the patients or users. Various information may also be collected through various automated or manual data collection means. The created database contains data on the user's past or historical sharps deposit behaviour including whether each past deposit is made on time, early, late or missed. The created database also contains information relating to each user's prescription including recommended or scheduled medical regimen. The database 104 may further contain information such as user location, user mobile phone number, and/or SMS time, etc. The database 104 may further contain information on user age, gender, diagnosis, etc. This database can also be created by use of other data sources such as medical records or the like that are not uniquely associated with any particular sharps bin.

The generated adherence database is saved in a storage device such as a cloud-based server and is accessible, in accordance with the present teaching, for making predictions. The adherence database may be operatively linked to, or accessible by, the one or more sharps bins to receive and save data collected from the one or more sharps bins. The database may also be operatively linked to other computing devices such as personal computers, personal assistants, and/or mobile phones for transmitting and/or receiving data. The database may be continuously updated to include more information on existing users or information on new users. The database may be multiple databases linked through a network.

### Pre-processing data

In accordance with the present teaching, the data within the adherence database is used to train a predictive model. Before training the predictive model, raw data is extracted from the database and is pre-processed at 304 for further use. According to some embodiments of the invention, data associated with one or more scenarios which are known to cause anomalous record of behaviour is excluded. For example, data associated with one or more loading doses, accidental trigger of patient monitoring units, and/or data collected from a patient monitoring unit which had a status of "inactivity" during a specific time period (i.e. no activity for more than 90 days) can be excluded from being used for model training. A loading dose is an initial higher dose of medication that may be given at the beginning of a course of treatment before dropping down to a lower maintenance dose. Accordingly, a loading dose may involve a larger number of or more frequent deposits of sharps than those scheduled in the regimen. In some embodiments, the entire dataset may be split into two subsets: training set and validation set.

Subsequent to data pre-processing, feature engineering is performed at 306. In particular, information gain for each of the features available within the data set (i.e. gender, age, user indication, etc.) is computed. One or more highest ranked features are selected to train the model.

Once the features are finalised, data required to train the model is transformed into a numerical format using, for example, normalisation and aggregation techniques at 308.

In the event where the predictive model is an ensemble model which is constituted from a plurality of individual base models, before training the ensemble model, all of the individual base models are cross validated and tuned at 310 so that the individual base models are generalised for predictions.

An exemplary data extraction and pre-processing process may be described as follows according to some exemplary embodiments of the present invention. The adherence platform includes a wide range of information regarding patients and their treatment programme, from the scheduled medication date and frequency of each patient to the drop status of scheduled injections. The dataset used for training the predictive machine learning models was collected over three years. The dataset contains information from 4,609 patients and 133,210 individual drop events. The data is retrieved and exported from a database. The data may be in the format of CSV files for further processing. After obtaining the dataset, personal data such as gender, age, name and address was removed in order to comply with EU General Data Protection Regulation (GDPR) policies. The dataset is then cleaned to remove empty rows/columns and redundant variables. Any missing data is imputed as median. Categorical data other than drop status is transformed into a machine learning-compatible format using one hot encoding. The one hot encoding is a classic technique applied in machine learning that converts categorical variables into a form that is suitable for provision to machine learning algorithms. The time stamp of the drop status a converted to labels as "On-Time" / "Not On-time" based on the time difference between the drop time stamp and the scheduled medication time stamp. In some embodiments, drop status labels are defined as follows.
- On-Time: If the medication was taken 24 hours earlier or later than the specified medication scheduled time.
- Early: If the medication was taken 48 hours to 72 hours earlier than the specified medication scheduled time.
- Late: If the medication was taken 48 hours to 72 hours later than the specified medication scheduled time.
- Missed: If the medication was taken outside the time frame provided above or not taken at all.

In some embodiments, the categories of "On-Time", "Early" and "Late" are defined as "On-Time" as the drops occurred during a specified time frame, while "Missed" is labelled as "Not On-time". In this way, the classification can be carried out as a binary classification.

It should be noted that the timeframes used in the above drop status labels are exemplary timeframes. Depending on the drug regimen and/or patient conditions, other suitable timeframes may be used to label drop status.

### Training model

A predictive model may be trained in an end-to-end fashion using the data collected by the medicament containers. For ensemble methods to be more accurate than any of its individual base models operating and predicting in isolation, the base models or base learners must be as accurate as possible and as diverse as possible. To achieve this, the base models are tuned relative to one another to ensure more accurate results. In preferred implementations all of the pre-processed data is used to train each base model at 312. To build a high-performance or robust ensemble model, a subset of all the trained base models containing the most correlated trained base models are selected to build the ensemble model. For example, as shown in Figure 1B, the totality of the trained models is evaluated and five of what are identified as being the most correlated models among the seven trained models are selected to build the ensemble. It should be noted that although typically an ensemble consisting of the most correlated base models provides the best overall predictive performance, it may be the case that an ensemble comprising some base models with inferior levels of correlations may provide a superior overall predictive performance and thus may be selected to build the ensemble predictive model. The trained predictive model is saved in a storage device such as a cloud-based server and is accessible by a cloud-based adherence service for making predictions.

Trained predictive models are tested on real world self-medicating patient data from an unseen dataset to ensure good prediction performance and generalisation.

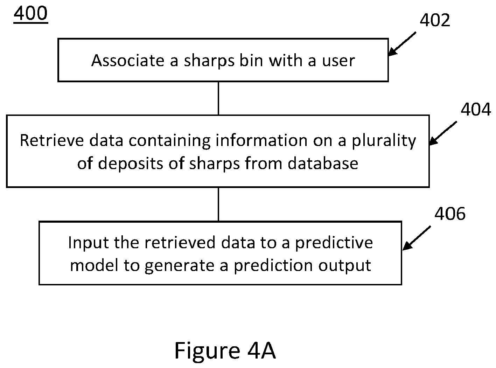

Patients' adherence is predicted by leveraging the predictive models based on the real time data collected from the medicament containers. Predictions are made on a rolling window for each patient drug regime. Figure 4A shows a flowchart illustrating a method 400 of predicting a user's future adherence behaviour. By way of illustration, a patient who is prescribed with a specific injection schedule and wishes to use the adherence service is firstly associated with a specific sharps bin at 402. The association may be completed by linking the sharps bin 108 with the user's one or more computer devices 110 such as personal computers, personal assistants, and/or mobile phones using Bluetooth device, NFC device, bar code, QR code, RFID tag, and/or other devices. Once the sharps bin 108 is associated with the user, data captured for the user via the sharps bin 108 can be stored in the corresponding fields of the lookup table 120. The adherence platform 102, also referred to as adherence service or service, may retrieve data of the user through the lookup table 120 for making predictions. In the embodiment where the sharps bin is configured to support multiple users, each user can create a respective account which is associated with the sharps bin in a similar manner.

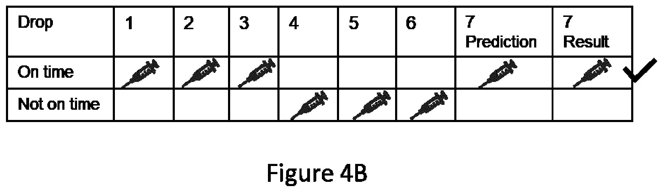

To make a prediction, a "prediction input file" is auto-generated by retrieving relevant data from the adherence database. A set of data containing information on a plurality of past deposits of sharps made by the user is retrieved at 404. In one embodiment, the retrieved data is a sequence of time series data containing information on a plurality of deposits of sharps made by the user in the past. For example, as shown in Figure 4B, adherence data on the user's past six deposits of sharps is retrieved from the adherence database to form at least part of the prediction input file. In some embodiments, the retrieved data contains information on a list of all drops or deposits scheduled for a specific day. In some other embodiments, the retrieved data contains one or more features of the user as described in the process of feature engineering.

The retrieved set of data is inputted into the predictive model to generate a prediction output at 406. The prediction output contains information on whether an anticipated deposit of at least one sharp by the user will be made on time. In one embodiment, the prediction output indicates whether an anticipated deposit of at least one sharp would be made by the user within a scheduled deposit timeframe. A classification engine 101 may be used to classify the sharp deposit information received from a sharps bin associated with a user. The classification engine 101 may be configured to compare the timestamp of the medicament information to that of the prescribed medicament schedule and accordingly classify the medicament information. For example, a sharps deposit made according to a medicament schedule, for example, within a scheduled deposit timeframe is considered and classified as "good" or "on time". A sharps deposit made outside of the scheduled deposit timeframe is considered to deviate from the schedule and classified as "bad" or "not on time". A not-on-time deposit may be classified as "early" if a sharps deposit is made before the timeframe. Similarly, a sharps deposit made after the timeframe may be considered and classified as "late". If a deposit is not made at all or made after another timeframe, the deposit is classified as "missed". Sharps deposits classified as "early", "late", or "missed" may be considered as deviating from the timeframe specified in the user's prescribed sharps deposit schedule. For example, patients may typically have 72 hours from their scheduled time to administer their medication. If a deposit is made after 72 hours from the scheduled time or not made at all, it is classified as "missed". With reference to Figure 4B where the prediction input file comprises information on the user's past six deposits, the prediction output indicates whether the user is going to make an anticipated seventh deposit on time. In general, the last n deposits or drops opportunities information is used to predict the n+1th deposit or drop opportunity. In some embodiments, instead of a single anticipated deposit, a prediction output may contain information on whether a time series of anticipated deposits over a specific time period will be made on time. For example, two injections may be scheduled for a single day. In this event, the prediction output may contain information on whether the user will make the two anticipated deposits scheduled for that day on time.

According to some embodiments, the prediction output may indicate whether an anticipated deposit of at least one sharp will be made on time. According to some other embodiments, the prediction output may contain information on whether an anticipated deposit of at least one sharp will be made on time, early, late, or missed. A deposit within a scheduled deposit timeframe is considered and classified as "on time". A deposit before the timeframe is classified as "early". A deposit after the timeframe is considered as "late". If the deposit is made after a maximum acceptable time frame for administering the medication, or not made at all, it is classified as "missed". According to some embodiments, the prediction output may further contain information on the probability of the anticipated deposit of at least one deposit being made on time, early, late, and/or missed. For example, a prediction may indicate that a user is likely to make the next deposit on time with a probability of 80% and there is a 20% probability that the user may not make the next deposit on time. The prediction output may be indicative of a probability and/or a score of the expected sharp deposit being made on time. The probability of the prediction may be further improved by considering user's physiological parameters. For example, a heart monitor may be employed to measure the user's heart rate. The measured heart rate may be used to improve the probability of the prediction.

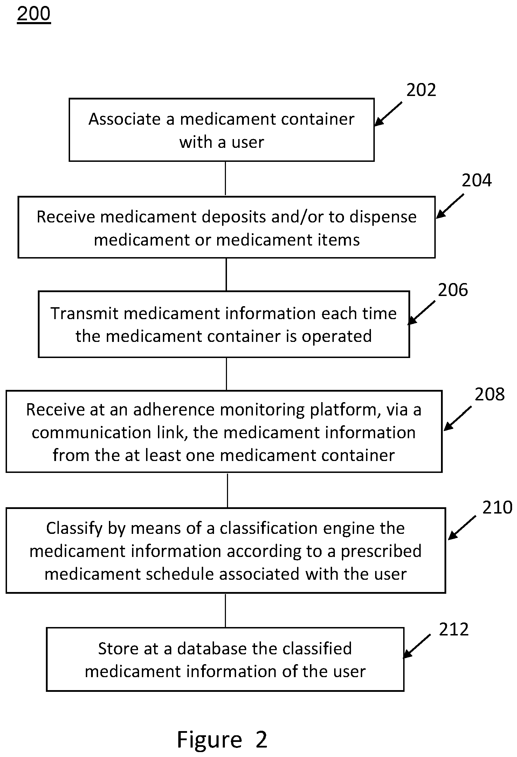

In the embodiments where the predictive model is an ensemble predictive model, the prediction input file is inputted into each of the base models of the ensemble. Each base model generates a respective prediction output. In the embodiment shown in Figure 1B, majority voting is used to select a final prediction based on the prediction output generated by each base model. In particular, as shown in Figure 2 where five base models are used to form the ensemble predictive model, each base model generates a respective prediction output indicating whether the next deposit will be made on time. As the majority of the prediction output in this case is "not on time", the final prediction output is determined to be "not on time". Although illustrated using the example of majority voting, the skilled person would understand that other suitable algorithms may be employed to determine the final prediction based on the base model predictions.

In the embodiments where the prediction output further contains information on probability, a final prediction may indicate that the user is likely to make the next deposit on time with a probability of 80% and there is a 20% probability that the user may not make the next deposit on time.

### Intervention

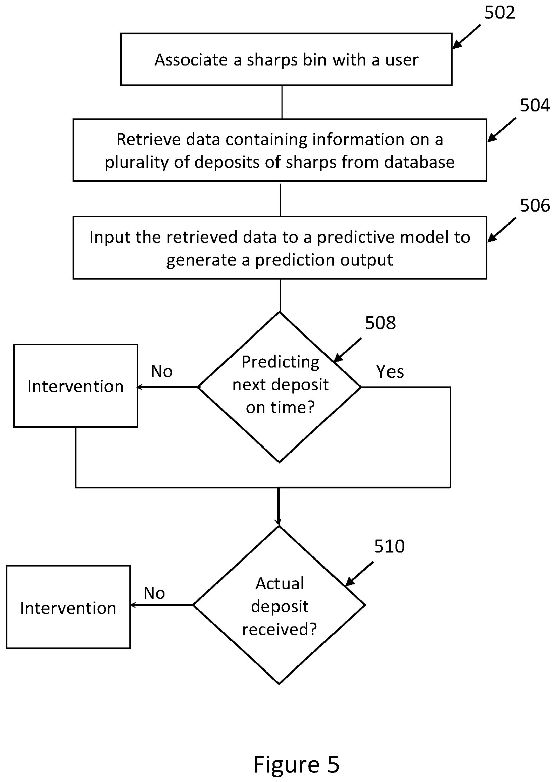

Based on the prediction output generated at 406, the adherence service may initiate various communications and/or actions in order to assist the user to adhere to their medical regimen. Figure 5 shows a flow chart illustrating an embodiment where the adherence service 102 initiates interventions based on the prediction output according to an embodiment of the present invention. Steps 502, 504, and 506 of Figure 5 are substantially the same as steps 402, 404, and 406 described in connection with Figure 4. sharps bin is associated with a user at 502. Data containing information on a plurality of deposits of sharps is retrieve from database at 504. The retrieved data is inputted to a predictive model to generate a prediction output at 506.

At 508, in response to a prediction that the user will not make their next, expected deposit on time (within a scheduled deposit timeframe), the adherence service 102 may trigger and issue an event to prompt the user that according to their prescribed sharps deposit schedule an sharps deposit is expected . Based on the prediction result indicated by the prediction output, the event may be a communication to the user of the sharps bin. The communication may be an alert, a reminder, and/or one or more intervention actions. For example, if the predication output indicates that the user will not make the next deposit on time, a reminder or an alter will be sent before the scheduled injection or deposit timeframe. The communication may also be an invitation to the user's family member or healthcare provider for manual intervention. The communication may also be a message setting out the predicted result. The communication may be sent from the adherence server to the one or more user device 110 and/or the sharps bin 108 where the message is displayed. It will be appreciated that these variances in type of communications and routing of same can be uniquely tailored or programmed for individual users. For example, the message may be an SMS message sent to the user's mobile phone or could be a telephone message (which could be automated in nature) sent to an associate of the user. The communication may take various forms and contain various information and may be sent to one or more devices of the user including the sharps bin. For example, the communication may be an SMS message sent to the user's mobile phone 110. The SMS message may simply show the prediction result. More typically, the communication is an alert sent to the user to remind the user to take the scheduled injection. The alert may indicate a scheduled time or timeframe for making the injection, the name of the medication, the required dose of the medication, and/or a brief instruction on how to correctly make the injection, etc. Similar interventions may be initiated in response to predicting that the next deposit will be made early or not made at all (missed). In the event that an "early" deposit prediction is made, the adherence service may trigger an intervention prior to a specific timeframe to prevent the anticipated early deposit. In the event that a "missed" deposit is predicted, different levels of interventions may be triggered. For example, the adherence service 102 may initiate more than one reminder prior to the scheduled time of the next injection.

In response to a prediction that the user will make the next deposit on time, the adherence service 102 typically takes no action and wait for the user to make the scheduled injection and sharp deposit. Although typically no action is taken in response to an "on time" prediction, a communication to the user may nevertheless be triggered (not shown in the figure) to present certain information such as a mere indication of the predicted result. Although described using the user's mobile phone, it should be noted that the communication may be sent to any alerting device such as a designated pager (not shown) or a designated user's device e.g. a caregiver, or a family member, and the like. For example, a sudden abnormality in the adherence pattern (e.g. sharps deposit was not received within a user's regular deposit time) may indicate that there is something wrong with the patient e.g. they are unwell and may need assistance. Therefore, in the case of a detection of an abnormal behaviour, the system may issue an alert signal to a registered user of the adherence platform such as a nurse, a family member, and the like.

According to some embodiments, depending on the predicted probability that the user may not make the next deposit on time, the adherence service 102 may initiate various levels of alert or reminder prior to the scheduled timeframe of making the next injection and the corresponding sharp deposit.

According to some embodiments, depending on whether the user responds to the alert, further interventions may be issued. For instance, if no response from the user has been received acknowledging receipt of the alert, further alert or an alternative form of intervention may be triggered.

At 510, the sharps bin may be configured to detect one or more actual deposits of sharps to confirm receipt of the at least one anticipated sharp deposit. An actual deposit is a term used herein to reflect an actual receipt of a physical sharps within the sharps bin. This may be detected using the one or more sensors 140 that are provided as functional components of the sharps bin. A time of the detected actual deposit of the at least one sharp is recorded. As a deposit of a sharp is typically made immediately following administering the medication. The time of deposit can be considered as the time of administration of the medication. However, it may be desirable to consider the time gap between the two actions by introducing a time offset. In either case, the time-stamped information may be stored locally on the sharps bin or transmitted to a remote storage device. The scheduled time of making a deposit is generally in line with a time schedule of administration of medication of the user.

Such a detection of one or more actual deposits can be used to confirm that an anticipated deposit of at least one sharp has been made. According to an embodiment of the present invention, subsequent to issuing the communication, depending on whether an actual deposit is detected within a specific time frame, further intervention may be initiated. For example, if no confirmation that an actual deposit corresponding to the anticipated deposit has been received at or immediately after the scheduled time or timeframe, one or more further alerts or reminders may be issued to remind the user to make an injection so as to conform with their regimen. In the absence of confirmation of receiving an actual deposit within a further time period after the scheduled timeframe, other forms of intervention action may be triggered. For example, a communication to a family member or healthcare provider is triggered to invite human intervention.

According to an embodiment of the invention, a deposit confirmation may be carried out by generating an adherence data indicating whether the detected actual deposit of at least one sharp is made within the scheduled deposit timeframe (on time) by comparing the timestamped actual deposit of at least one sharp with the scheduled deposit timeframe. The adherence database may be updated to include this adherence data.

According to an embodiment of the invention, the one or more cameras 150 in the sharps bin may capture an image of the detected at least one sharp. The captured image may be sent to the database. The captured image may be sent to the adherence service 102 for further analysis. The service may comprise an image recognition unit configured for receiving the image of the sharps deposit, performing image analysis on the image of the sharps deposit, and determining, based on the image analysis, whether the detected sharps deposit is a valid sharps deposit. For example, the image recognition unit may be configured to analyse the image received from the sharps bin to extract a set of features e.g. shape, name, colour, and the like. The features may be extracted and classified using a known feature extraction algorithm. The image recognition unit may assess the validity of the sharps deposit by comparing the extracted features to the features of the expected sharps deposit, and accordingly assess the validity of the sharps deposit.

### Validation

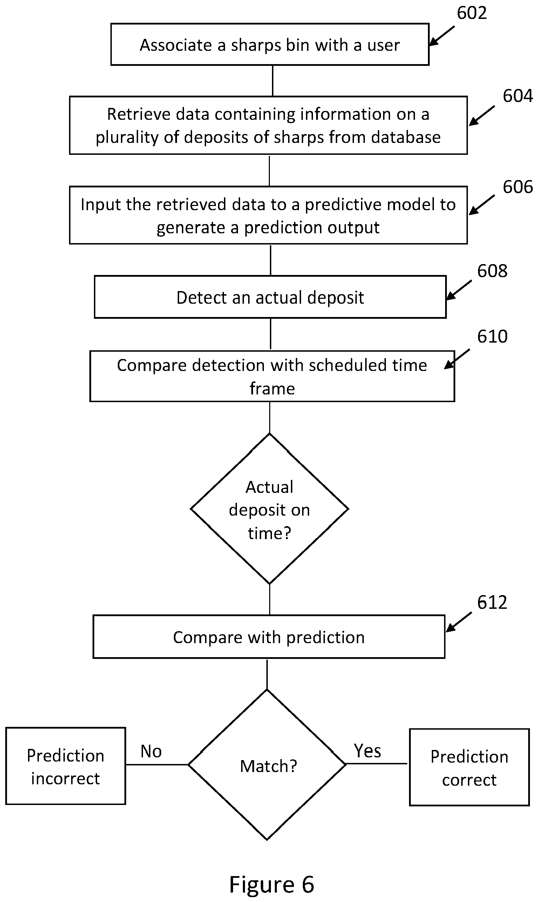

According to an embodiment of the present invention, a prediction made by the predictive model is validated by comparing the prediction output with a detected actual deposit of a sharp made by the user. Figure 6 is a flowchart illustrating a process of validating an adherence system in accordance with an embodiment of the present invention. Steps 602, 604, and 606 are substantially the same as steps 402, 404, and 406 of Figure 4 and are not described herewith in detail. At 602, a sharps bin is associated with a user. Data containing information on a plurality of deposits of sharps is retrieve from database at 604. The retrieved data is inputted to a predictive model to generate a prediction output at 606. Subsequent to generating the prediction output, one or more sensors 140 located in the sharps bin may be used to detect an actual deposit of at least one sharp into the sharps bin. The time of the detected actual deposit of the at least one sharp is recorded by for example, timestamping, by means of a timestamping engine, the received sharp deposit as aforementioned. The timestamped actual deposit is compared with a scheduled time or timeframe for making a deposit of at least one sharp to determine whether the detected actual deposit has been made on time (not shown). For example, if the deposit is made within a scheduled timeframe, it is classified as "on time". if the deposit is made before the scheduled timeframe, it is classified as "early". If the deposit is made after this time frame, it is classified as "late". If the deposit is made after a maximum acceptable time frame for administering the medication, or not made at all, it is classified as "missed". The specific timeframes may be selected based on the prescribed medication. For example, patients typically may have 72 hours from their scheduled time to administer their medication. If a deposit is made after 72 hours from the scheduled time or not made at all, it is classified as "missed".

Based on the compared result, a validation input data is generated. The validation input data may correspond to the adherence data of the deposit confirmation step. The validation input data contains information on whether the detected actual deposit is on time. According to an embodiment of the present invention, a validation output is generated based on comparing the validation input data with the scheduled deposit timeframe at 610. At 612, the generated validation input data is inputted to a comparing module to compare with the prediction output generated to generate a validation output. If the validation data matches the prediction output, the prediction is validated as correct. If the validation data does not match the prediction output, the prediction is considered to be incorrect. The validation data may be used to train the predictive algorithm.

According to another embodiment of the present invention, a validation is made for a series of predictions made for a specific day. In this embodiment, the validation file contains data representing whether each of a corresponding series actual deposits detected during that day is on time. This generated validation file is then compared with the series of predictions made for that day. Such a validation file may be generated daily and the validation process may be repeated daily. For example, a prediction of whether a deposit scheduled for 4 November will be made on time is made on 1 November. This prediction may then be validated using a validation file generated on 4 November. A prediction made on 10 December may be validated on 13 December.

According to an embodiment, in order to improve accuracy of prediction, validation results are excluded under certain circumstances. Such circumstances include but are not limited to: a) where a deposit in a validation file is self-reported; b) where the user's sharps bin has been unplugged for more than 30 days; c) where the user has deactivated the sharps bin between the time of making a prediction and the time of performing a validation of the prediction; and/or d) where a deposit being predicted is a loading dose. This is because deposits that are classified as a loading dose are excluded when training the predictive model as described in the model training process.

According to an embodiment of the present invention, the generated validation file is added to the adherence database and is accessible by the predictive model for making future predictions.

According to an embodiment of the present invention, to make another prediction, a second "prediction input file" is auto-generated by retrieving a second set of data from the adherence database. The retrieved second set of data contains information on a plurality of deposits of sharps made by the user in the past. In one specific embodiment, the retrieved second set of data contains the validation input data added to the database. For example, data containing information on the user's past seven deposits of sharps is retrieved from the adherence database to form the prediction input file. In one embodiment, the retrieved second set of data contains the validation input data added to the adherence database. For example, the validation data generated on 4 November may be added to the adherence database. This validation data may be later retrieved as part of a set of data forming a prediction input file for making predictions.

In other embodiments, the retrieved second set of data further contains information on a list of all drops or deposits scheduled for a specific day. In some further embodiments, the retrieved second data further contains one or more features of the user as already described in the process of feature engineering. The retrieved second set of data is inputted to the predictive model to generate a second prediction output. The second prediction output contains information on whether a second anticipated deposit of at least one sharp by the user will be made on time.

According to some embodiments, model validation, evaluation and/or testing can be carried out as set out below. Drop data is recorded by sharps bins and uploaded to the data server for storage on a daily basis. This daily generated dataset is retrieved for a period of circa three months and used as the testing dataset, as using data previously unseen by the models means that the testing work is free from "data leak" problems. During this period, for patients expected to take medication, the "prediction file" or prediction input file containing the historic drop information of the six most recent drops is collected at the beginning of the scheduled medication day. This data is used to predict if the 7th (next) drop would be "on-time" or "not on-time". Once the "prediction file" has been extracted, it is fed to the proposed machine learning models in order to predict which category of classification ("on-time" / "not on-time') would be allocated to the patient that day. As drops made 72 hours before or after the scheduled medication time are labelled as "on-time", in order to give patients enough time to take medication and dispose of the used injector, a "testing file" (including the ground truth on the real behaviours of patients) is collected 72 hours after the scheduled medication day. The testing file may correspond to a file containing validation input data. The testing work compared the "prediction file" with the "testing file". For example, a patient is scheduled to take medication on day 5 January. The "prediction file" is generated at 12 am on 5 January and contains data from the last 6 drop. The "testing file" with the real-world actual drop result is then collected at 12 am on 8 January. During the period from 12 am on 2 January to 12 am on 8 January, if the patient takes the medication and makes the right disposal within 144 hours, the behaviour can be labelled as "on-time", otherwise, the drop is classified as "not on-time". This data was recorded in "testing file" as ground truth for further testing work. Before conducting the testing work, data generated in the following scenarios is removed:
- Unplugged: if the patient's unit is unplugged for a period of more than 30 days before the scheduled medication drop day.
- Deactivated: if the patient's unit is disconnected in the period between extraction of the "prediction file" and "testing file" (excluded from the dataset due to insufficient knowledge regarding the ground truth of the patient's test drop).
- Self-Reported: if the patient finds the drop information to be incorrect, they can report their drop status independently after the scheduled drop time (e.g. up to 20 days after the event). Inclusion of self-reported drops/amended drops are not suitable as there is too much uncertainty in patient behaviour and accurate drop information is considered as the ground truth for testing.
- Loading Dose: loading doses were excluded as no drops labelled as a loading dose were included in the training set.

The systems and methods of the described embodiments may be implemented in a computer program product such as a physical, non-transitory computer readable medium having computer readable instructions. The medium may be provided in various forms, including one or more diskettes, compact disks, tapes, chips, magnetic and electronic storage media, volatile memory, non-volatile memory and the like. Non-transitory computer-readable media may include all computer-readable media, with the exception being a transitory, propagating signal. The term non-transitory is not intended to exclude computer readable media such as primary memory, volatile memory, RAM and so on, where the data stored thereon may only be temporarily stored. The computer useable instructions may also be in various forms, including compiled and uncompiled code.

The embodiments of the systems and methods described herein may be implemented in hardware or software, or a combination of both. These embodiments may be implemented in computer programs executing on programmable computers, each computer including at least one processor, a data storage system (including volatile memory or non-volatile memory or other data storage elements or a combination thereof), and at least one communication interface. For example, and without limitation, the various programmable computers may be a server, network appliance, set-top box, embedded device, computer expansion module, personal computer, laptop, personal data assistant, cellular telephone, smartphone device, UMPC tablets and wireless hypermedia device or any other computing device capable of being configured to carry out the methods described herein.

Program code is applied to input data to perform the functions described herein and to generate output information. The output information is applied to one or more output devices, in known fashion. In some embodiments, the communication interface may be a network communication interface. In embodiments in which elements of the invention are combined, the communication interface may be a software communication interface, such as those for inter-process communication. In still other embodiments, there may be a combination of communication interfaces implemented as hardware, software, and combination thereof.

Each program may be implemented in a high level procedural or object oriented programming or scripting language, or a combination thereof, to communicate with a computer system. However, alternatively the programs may be implemented in assembly or machine language, if desired. The language may be a compiled or interpreted language. Each such computer program may be stored on a storage media or a device (e.g., ROM, magnetic disk, optical disc), readable by a general or special purpose programmable computer, for configuring and operating the computer when the storage media or device is read by the computer to perform the procedures described herein. Embodiments of the system may also be considered to be implemented as a non-transitory computer-readable storage medium, configured with a computer program, where the storage medium so configured causes a computer to operate in a specific and predefined manner to perform the functions described herein.

Furthermore, the systems and methods of the described embodiments are capable of being distributed in a computer program product including a physical, non-transitory computer readable medium that bears computer usable instructions for one or more processors. The medium may be provided in various forms, including one or more diskettes, compact disks, tapes, chips, magnetic and electronic storage media, volatile memory, non-volatile memory and the like. Non-transitory computer-readable media may include all computer-readable media, with the exception being a transitory, propagating signal. The term non-transitory is not intended to exclude computer readable media such as primary memory, volatile memory, RAM and so on, where the data stored thereon may only be temporarily stored. The computer useable instructions may also be in various forms, including compiled and non-compiled code.

The words comprises/comprising when used in this specification are to specify the presence of stated features, integers, steps or components but does not preclude the presence or addition of one or more other features, integers, steps, components or groups thereof.

## Claims

1. A system (100) for monitoring patient medical adherence behaviour, the system comprising:
at least one medicament container (108) associated with a user, the medicament container (108) being configured to receive medicament deposits and/or dispense medicament, and to transmit medicament information each time the medicament container (108) is operated by the user; and
a medical adherence monitoring platform (102) configured for at least receiving, via a communication link, medicament information from the at least one medicament container (108), the adherence monitoring platform (102) comprising:
a classification engine (101) configured for classifying the medicament information according to a prescribed medicament schedule and/or medicament dosage regime associated with the user, the classified medicament information being indicative of the medical adherence of the user to their prescribed medicament schedule and/or medicament dosage regime; and
an adherence database (104) configured for storing the classified medicament information of the user.

2. A system according to claim 1, wherein the medical adherence monitoring platform is configured for receiving medicament information from at least one smart drug administering system (120), and/or at least one smart monitoring device (130).

3. A system according to claim 1 or claim 2, wherein the medicament information at least comprising information on medicament deposits or dispensations, and/or information on user weight parameter, and/or medicament specific information, and/or information extracted from a blood sample.

4. A system according to any one of the preceding claims, wherein the adherence monitoring platform (102) comprising:
a predictive model (106) trained using the classified medicament information stored in the database (104); and
at least one processor configured to analyse the medical adherence behaviour of the user by:
retrieving (404, 504, 604) from the database (104) classified medicament information associated with the user; and
inputting (406, 506, 606) the retrieved classified medicament information to a predictive model (106) to generate a prediction output indicating whether an expected medicament deposit or dispensation would be made by the user within a timeframe specified in their medicament schedule and/or corresponds to a predetermined medicament dosage regime; wherein the processor is configured for issuing a first communication to at least one user device (110) when the prediction output indicates a deviation from the user's medicament schedule and/or medicament dosage regime.

5. A system according to any one of the preceding claims, wherein the medicament container (108) is a sharps bin configured for receiving sharps deposits, the sharps bin (108) comprising:
a housing configured to receive sharps deposits;
at least one sensor module (140) configured to detect a sharps deposit;
a timestamping module configured to timestamp the detected actual deposit of at least one sharp; and
a communication module (160) configured for transmitting sharps deposit information to the adherence monitoring platform (102).

6. A system according to claim 5, wherein the at least one sensor module comprises a camera (150) configured for capturing an image of the sharps deposit, wherein the sharps deposit information comprising an image of the sharps deposit and associated timestamping information.

7. A system according to claim 6, wherein the medical adherence monitoring platform (102) comprising an image recognition unit configured for:
receiving the image of the sharps deposit;
performing image analysis on the image of the sharps deposit;
determining, based on the image analysis, whether the detected sharps deposit is a valid sharps deposit; and
issuing a second communication to the at least one user device (110) based on determining that the detected sharps deposit is not a valid sharps deposit.

8. A system according to any one of claims 5 to 7, wherein the classification engine (101) is configured for comparing the timestamping information of each sharps deposit with the timeframe indicated in the prescribed sharps deposit schedule to classify, based on the comparison results, whether the sharps deposit is on time.

9. A system according to claims 3 to 8, wherein the classification engine (101) is configured for classifying medicament deposits or dispensations as overdosing to indicate excessive use of the medicament, and/or underdosing to indicate underdosing of the medicament.

10. A system according to any one of claims 4 to 9, wherein the processor is configured to issue the first communication before the timeframe indicated in the prescribed medicament schedule.

11. A system according to any one of claims 4 to 10, wherein the processor is configured to issue a third communication to the user in the absence of a medicament information confirming that the expected sharp was made within the medicament schedule timeframe, and/or the medicament dosage regime is correctly adjusted.

12. A system according to any one of claims 4 to 11 wherein the first and/or third communication is an alert, a reminder, and/or an intervention action.

13. A system according to any of the claims 4 to 12, wherein the prediction output is indicative of a probability and/or a score of the expected sharp deposit being made on time, and/or a dose corresponding to the prescribed dosage regime.

14. A system according to any one of the claims 4 to 13, wherein training the predictive model (106) comprises the steps of:
retrieving (302) classified sharps deposit information from the database;
pre-processing (304) the retrieved classified sharps deposit information by omitting anomalous data associated with anomalous sharps deposit information, wherein the anomalous sharps deposit information comprises:
loading doses;
accidentally triggered deposits; and/or
data collected from a sharps bin that has been inactive for a predefined period of time.

15. A method for monitoring patient medical adherence, the method comprising the steps of:
providing (202) at least one medicament container (108) associated with a user, the medicament container (108) being configured to receive medicament deposits and/or to dispense medicament, and to transmit medicament information each time the medicament container (108) is operated by the user; and
receiving (204) at an adherence monitoring platform (102), via a communication link, the medicament information from the at least one medicament container (108), the adherence monitoring platform (102) being configured to perform the steps of:
classifying (210) by mean of a classification engine (101) the medicament information according to a prescribed medicament schedule associated with the user and/or dosage regime, the classified medicament information being indicative of the medical adherence of the user to their prescribed medicament schedule and/or dosage regime; and
storing (212) at a database (104) the classified medicament information of the user.
